# EUROPEAN PATENT SPECIFICATION

(11) **EP 3 629 964 B1**
(45) Date of publication and mention of the grant of the patent: **25.09.2024**
(21) Application number: 18770113.1
(22) Date of filing: 22.05.2018
(51) Int. Cl.: A61B 18/04, A61N 1/36

(54) **LESION MATURATION PREDICTION USING LOCAL IMPEDANCE**
LÄSIONSREIFUNGSVORHERSAGE UNTER VERWENDUNG VON LOKALER IMPEDANZ
PRÉDICTION DE MATURATION DE LÉSION À L'AIDE D'UNE IMPÉDANCE LOCALE

(30) Priority: 23.05.2017 US 201762510258 P
(43) Date of publication of application: 08.04.2020
(73) Proprietor: Boston Scientific Scimed Inc., Maple Grove, Minnesota 55311 (US)
(72) Inventor: SULKIN, Matthew, S., New Brighton MN 55112 (US); LAUGHNER, Jacob, I., Medford, MA02155 (US); SHUROS, Allan, C., St. Paul MN 55116 (US); BYRON, Mary, M., Roseville MN 55113 (US); HAMANN, Jason J., Blaine MN 55449 (US)
(74) Representative: Pfenning, Meinig & Partner mbB
(86) International application number: PCT/US2018/033950
(87) International publication number: WO 2018/217798

(56) References cited:
- WO-A1-2016/054379
- WO-A1-2017/151576
- US-A1- 2002 068 931
- US-A1- 2012 143 177

## Description

### CROSS-REFERENCE TO RELATED APPLICATION

This application claims priority to Provisional Application No. 62/510,258, filed May 23, 2017.

### TECHNICAL FIELD

The present disclosure relates to therapies for cardiac conditions. More particularly, the present disclosure relates to methods and systems for ablation of cardiac tissue for treating cardiac arrhythmias.

### BACKGROUND

US 2002/068931 A1 relates to an apparatus for ablating organic material, such as tissue. Inorganic materials, such as plastic, can be analyzed, welded or otherwise acted upon and a monitor signal will provide indicia representative of real time characteristics of the material and change of character thereof. Application of the monitor signal alone can be used to determine the real time characteristics of either organic or inorganic materials to distinguish between healthy and abnormal or diseased tissue and to distinguish between similar inorganic materials having been subjected to different environments or conditions.

US 2012/143177 A1 relates to a device for ablating tissue in a body organ chamber, including heart chambers, comprising a guide body sheath having a deflectable distal end portion; a guidewire extendable from said distal end portion of said guide body to form a guidewire loop that is controllable to create forced contact with organ chamber tissue; an inflatable ablation balloon device mounted on a flexible guide shaft able to travel over said guidewire; and a loop control element for controlling the size, shape and disposition of said guidewire loop.

WO 2016/054379 A1 relates to systems and methods for evaluating neuromodulation via hemodynamic responses. A system includes a neuromodulation catheter comprising an elongated shaft having a distal portion, and a plurality of electrodes spaced along a distal portion.

WO 2017/151576 A1 relates to a method for preventing esophageal injury during ablation of tissues in the heart. The method comprises applying, to a first electrode a first set of one or more electrical signals; measuring, from a second electrode, a second set of electrical signals resulting from the first set of one or more electrical signals being applied to the first electrode, wherein the second set of electrical signals characterizes an atrio-esophageal electric coupling between the first electrode located in a chamber of the heart and the second electrode located in a esophagus; and in response to the atrio-esophageal electric coupling, or a derivative parameter derived therefrom, satisfying an alert condition, causing an audible or visual alert to be generated.

Aberrant conductive pathways disrupt the normal path of the heart's electrical impulses. The aberrant conductive pathways can create abnormal, irregular, and sometimes life-threatening heart rhythms called arrhythmias. Ablation is one way of treating arrhythmias and restoring normal conduction. The aberrant pathways, and/or their sources, may be located or mapped using mapping electrodes situated in a desired location. After mapping, the clinician may ablate the aberrant tissue. In radio frequency (RF) ablation, RF energy may be directed from the ablation electrode through tissue to another electrode to ablate the tissue and form a lesion.

Lesion indexing provides the clinician feedback on the estimated lesion size during RF applications to improve both safety and efficacy of the procedure. Conventional lesion indexing algorithms typically utilize one or two parameters to estimate lesion size. These parameters include, for example, RF generator impedance (e.g., impedance measured between the RF electrode and a patch on the skin), RF dosing (power and duration), contact force and the force-time integral, catheter temperature, catheter interface temperature and catheter jump index. Lesion size estimation tends to be fairly limited and/or inaccurate with these conventional technologies. More robust algorithms include ultrasound and light scattering analyses, but these algorithms tend to be complex and computationally burdensome.

### SUMMARY

The invention is defined in claim 1. Any methods disclosed hereinafter do not form part of the scope of the invention and are mentioned for illustrative purposes only. Embodiments of the present invention facilitate real-time ablation lesion characteristic analysis. Electrodes are used to measure a local impedance based on an electrical signal (e.g., generated using electrodes), which may be, for example, a unipolar signal, a bipolar signal, and/or the like. In embodiments, an "electrical signal" may be, refer to, and/or include a signal detected by a single electrode (e.g., a unipolar signal), a signal detected by two or more electrodes (e.g., a bipolar signal), a plurality of signals detected by one or more electrodes, and/or the like. One or more local impedance metrics may be used to determine one or more lesion characteristics.

According to an example of the invention, an electrophysiology system, comprising: a catheter including: a flexible catheter body having a distal portion; and a plurality of electrodes disposed on the distal portion; the plurality of electrodes including: a radiofrequency (RF) ablation electrode; first second and third mapping electrodes positioned within RF ablation electrode; and a plurality of ring electrodes located proximally of the RF ablation electrode and including a first ring electrode located proximally to a distal end of the catheter, an intermediate second ring electrode positioned distally of the first ring electrode, and a distal ring electrode located distally of the second ring electrode; a signal generator configured to generate an electrical signal by driving one or more currents between a first set of the plurality of electrodes defined by the RF ablation electrode and the first ring electrode, wherein a second set of the plurality of electrodes defined by one of the mapping electrodes and the distal ring electrode is configured to obtain an impedance measurement based on the electrical signal; and a mapping processor configured to: receive the impedance measurement from the second set of electrodes; determine at least one impedance metric; and determine at least one lesion characteristic based on the at least one impedance metric.

Optionally, the first set of the plurality of electrodes may include at least one electrode that is not in the second set of the plurality of electrodes.

Optionally, the plurality of electrodes may further include at least one of a mapping electrode disposed on the distal portion of the catheter, an embedded electrode, and a printed electrode.

Optionally, the at least one lesion characteristic may comprise at least one of an existence of the lesion, a depth of the lesion, and/or a size of the lesion.

Optionally, the at least one impedance metric may comprise at least one of an initial impedance, an impedance drop, a derivative of an impedance signal over a period of time, and an integral of an impedance signal over time.

In an unclaimed Example that is provided for background information only, a method for determining a lesion characteristic using a catheter having a plurality of electrodes disposed on a distal end thereof, the method comprising: generating an electrical signal using a first set of the plurality of electrodes; measuring, using a second set of the plurality of electrodes, a local impedance based on the electrical signal; determining at least one local impedance metric; and determining at least one lesion characteristic based on the at least one impedance characteristic.

Optionally, the unclaimed method may further compriseproviding an indication associated with the at least one lesion characteristic.

Optionally, the at least one lesion characteristic may comprise at least one of an existence of the lesion, a depth of the lesion, and/or a size of the lesion.

Optionally, the at least one impedance metric may comprise at least one of an initial impedance, an impedance drop, a derivative of an impedance signal over a period of time, and an integral of an impedance signal over time.

Optionally, the first set of the plurality of electrodes may include at least one electrode that is not in the second set of the plurality of electrodes.

Optionally, the first set of the plurality of electrodes may comprise at least one ring electrode and an ablation electrode.

In an illustrative Example, an electrophysiology system, comprising: a catheter including: a flexible catheter body having a distal portion; and a plurality of electrodes disposed on the distal portion; a signal generator configured to generate an electrical signal by driving one or more currents between a first set of the plurality of electrodes, wherein a second set of the plurality of electrodes is configured to obtain an impedance measurement based on the electrical signal; and a mapping processor configured to: receive the impedance measurement from the second set of electrodes; determine at least one impedance metric; and determine at least one lesion characteristic based on the at least one impedance metric.

Optionally, the first set of the plurality of electrodes may include at least one electrode that is not in the second set of the plurality of electrodes.

Optionally, the plurality of electrodes may include a plurality of ring electrodes and an ablation electrode.

Optionally, the plurality of electrodes may further include at least one of a mapping electrode disposed on the distal portion of the catheter, an embedded electrode, and a printed electrode.

Optionally, the first set of the plurality of electrodes may comprise the at least one ring electrode.

Optionally, the first set of the plurality of electrodes may comprise a first ring electrode and the ablation electrode.

Optionally, the second set of the plurality of electrodes may comprise a second ring electrode.

Optionally, the second set of the plurality of electrodes may comprise the at least one mapping electrode.

Optionally, the at least one lesion characteristic may comprise at least one of an existence of the lesion, a depth of the lesion, and/or a size of the lesion.

Optionally, the at least one impedance metric may comprise at least one of an initial impedance, an impedance drop, a derivative of an impedance signal over a period of time, and an integral of an impedance signal over time.

Optionally, the mapping processor may beconfigured to utilize a classifier to determine the at least one lesion characteristic.

Optionally, the classifier configured to determine the at least one lesion characteristic based on the at least one impedance metric and at least one additional parameter.

Optionally, the at least one additional parameter may comprise at least one of a measure of catheter stability, a measure of RF generator impedance, an electrogram characteristic, an RF ablation energy power level, and an RF ablation energy delivery duration.

In an unclaimed Example that is provided for background information only, a method for determining a lesion characteristic using a catheter having a plurality of electrodes disposed on a distal end thereof, the method comprising: generating an electrical signal using a first set of the plurality of electrodes; measuring, using a second set of the plurality of electrodes, a local impedance based on the electrical signal; determining at least one local impedance metric; and determining at least one lesion characteristic based on the at least one impedance characteristic.

Optionally, further comprising at least one of providing an indication associated with the at least one lesion characteristic and automatically discontinuing delivery of RF ablation energy, in response to determining the at least one lesion characteristic.

Optionally, the at least one lesion characteristic may comprise at least one of an existence of the lesion, a depth of the lesion, and/or a size of the lesion.

Optionally, the at least one impedance metric may comprise at least one of an initial impedance, an impedance drop, a derivative of an impedance signal over a period of time, and an integral of an impedance signal over time.

Optionally, the first set of the plurality of electrodes may include at least one electrode that is not in the second set of the plurality of electrodes.

Optionally, the first set of the plurality of electrodes may comprise at least one ring electrode and an ablation electrode.

In an illustrative Example, an ablation system, comprising: an ablation catheter including: a flexible catheter body having a distal portion; and a plurality of electrodes disposed on the distal portion, the plurality of electrodes comprising a radio frequency (RF) ablation electrode and at least one ring electrode; a signal generator configured to generate an electrical signal by driving one or more currents between a first set of the plurality of electrodes, wherein a second set of the plurality of electrodes is configured to obtain an impedance measurement based on the electrical signal; and a mapping processor configured to: receive the impedance measurement from the second set of electrodes; determine at least one impedance metric; and determine at least one lesion characteristic based on the at least one impedance metric, the at least one lesion characteristic comprising at least one of an existence of a lesion, a lesion depth, and a lesion size.

While multiple embodiments are disclosed, still other embodiments of the presently disclosed subject matter will become apparent to those skilled in the art from the following detailed description, which shows and describes illustrative embodiments of the disclosed subject matter. Accordingly, the drawings and detailed description are to be regarded as illustrative in nature and not restrictive.

### BRIEF DESCRIPTION OF THE DRAWINGS

FIG. 1 is a schematic illustration of an ablation system, in accordance with embodiments of the subject matter described herein.
FIG. 2 is a block diagram depicting an illustrative mapping operating environment, in accordance with embodiments of the subject matter described herein.
FIG. 3 is a flow diagram depicting an illustrative unclaimed method of determining a lesion characteristic, in accordance with embodiments of the subject matter described herein.
FIGS. 4A-4C are schematic diagrams depicting illustrative electrode arrangements, FIG. 4A in accordance with the invention.

While the disclosed subject matter is amenable to various modifications and alternative forms, specific embodiments have been shown by way of example in the drawings and are described in detail below. The intention, however, is not to limit the subject matter disclosed herein to the particular embodiments described. On the contrary, the disclosure is intended to cover all modifications, equivalents, and alternatives falling within the scope of the subject matter disclosed herein, and as defined by the appended claims.

As used herein in association with values (e.g., terms of magnitude, measurement, and/or other degrees of qualitative and/or quantitative observations that are used herein with respect to characteristics (e.g., dimensions, measurements, attributes, components, etc.) and/or ranges thereof, of tangible things (e.g., products, inventory, etc.) and/or intangible things (e.g., data, electronic representations of currency, accounts, information, portions of things (e.g., percentages, fractions), calculations, data models, dynamic system models, algorithms, parameters, etc.), "about" and "approximately" may be used, interchangeably, to refer to a value, configuration, orientation, and/or other characteristic that is equal to (or the same as) the stated value, configuration, orientation, and/or other characteristic or equal to (or the same as) a value, configuration, orientation, and/or other characteristic that is reasonably close to the stated value, configuration, orientation, and/or other characteristic, but that may differ by a reasonably small amount such as will be understood, and readily ascertained, by individuals having ordinary skill in the relevant arts to be attributable to measurement error; differences in measurement and/or manufacturing equipment calibration; human error in reading and/or setting measurements; adjustments made to optimize performance and/or structural parameters in view of other measurements (e.g., measurements associated with other things); particular implementation scenarios; imprecise adjustment and/or manipulation of things, settings, and/or measurements by a person, a computing device, and/or a machine; system tolerances; control loops; machine-learning; foreseeable variations (e.g., statistically insignificant variations, chaotic variations, system and/or model instabilities, etc.); preferences; and/or the like.

Although the term "block" may be used herein to connote different elements illustratively employed, the term should not be interpreted as implying any requirement of, or particular order among or between, various blocks disclosed herein. Similarly, although illustrative methods may be represented by one or more drawings (e.g., flow diagrams, communication flows, etc.), the drawings should not be interpreted as implying any requirement of, or particular order among or between, various steps disclosed herein. However, certain embodiments may require certain steps and/or certain orders between certain steps, as may be explicitly described herein and/or as may be understood from the nature of the steps themselves (e.g., the performance of some steps may depend on the outcome of a previous step). Additionally, a "set," "subset," or "group" of items (e.g., inputs, algorithms, data values, etc.) may include one or more items, and, similarly, a subset or subgroup of items may include one or more items. A "plurality" means more than one.

As used herein, the term "based on" is not meant to be restrictive, but rather indicates that a determination, identification, prediction, calculation, and/or the like, is performed by using, at least, the term following "based on" as an input. For example, predicting an outcome based on a particular piece of information may additionally, or alternatively, base the same determination on another piece of information.

### DETAILED DESCRIPTION

Electrophysiologists may utilize any number of parameters to assess the formation and maturation of lesions on human tissue (e.g., cardiac tissue), which may include existence (e.g., whether a lesion has been formed), lesion size, lesion depth, likelihood of occurrence of a steam pop, and/or any number of other characteristics of an ablation lesion. In embodiments, parameters used to determine lesion characteristics may include physiological parameters (e.g., electrogram (EGM) morphology, EGM attenuation, etc.), device parameters (e.g., catheter stability, RF generator impedance, contact force, etc.), ablation parameters (e.g., RF dosing (RF power and duration of application), etc.).

Embodiments of the disclosure may be implemented using specialty catheters or catheters already commercially available. Embodiments include a hardware/software graphical user interface (GUI) used for acquiring electrical signals, performing sharpness analyses, and displaying the results during an ablation procedure. This may be accomplished, for example, with a stand-alone system or may be incorporated into existing systems such as the Bard LabSystem Pro or the Rhythmia Mapping System., both available from Boston Scientific Corporation of Marlborough, Massachusetts.

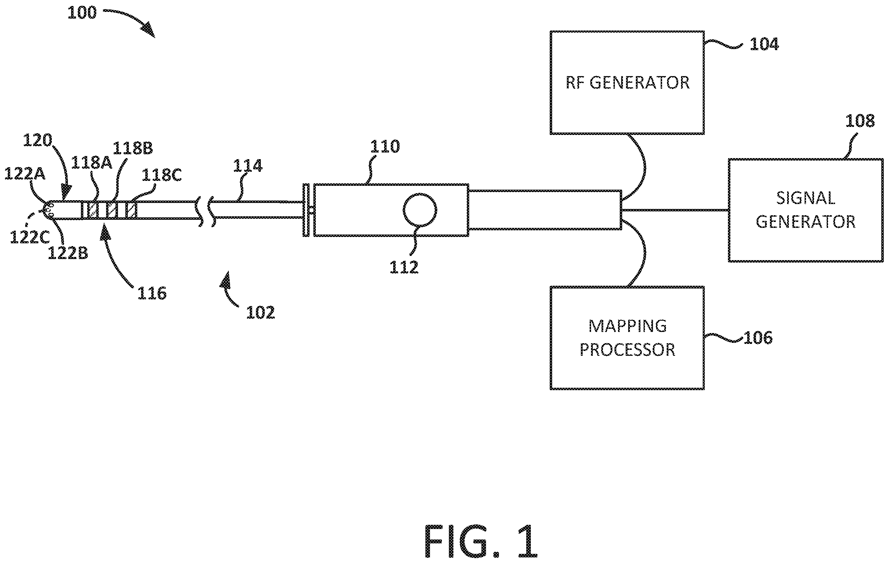

FIG. 1 is a schematic illustration of a radio frequency (RF) ablation system 100, in accordance with embodiments of the subject matter disclosed herein. As shown in FIG. 1, the system 100 includes an ablation catheter 102, an RF generator 104, a mapping processor 106, and a signal generator 108. The ablation catheter 102 is operatively coupled to the RF generator 104, the mapping processor 106, and the signal generator 108. As is further shown, the ablation catheter 102 includes a proximal handle 110 having an actuator 112 (e.g., a control knob, lever, or other actuator), a flexible body 114 having a distal portion 116 including a plurality of ring electrodes 118A, 118B, and 118C, a tissue ablation electrode 120, and a plurality of mapping electrodes 122A, 122B, and 122C (also referred to as "pin" electrodes or microelectrodes) disposed or otherwise positioned within and/or electrically isolated from the tissue ablation electrode 120. In various embodiments, the catheter system 100 may include other types of electrodes such as, for example, printed electrodes (not shown). In various embodiments, the catheter system 100 may also include noise artifact isolators (not shown), wherein the electrodes 122A, 122B, and 122C are electrically insulated from the exterior wall by the noise artifact isolators.

In some instances, the ablation system 100 may be utilized in ablation procedures on a patient and/or in ablation procedures on other objects. In various embodiments, the ablation catheter 102 may be configured to be introduced into or through the vasculature of a patient and/or into or through any other lumen or cavity. In an example, the ablation catheter 102 may be inserted through the vasculature of the patient and into one or more chambers of the patient's heart (e.g., a target area). When in the patient's vasculature or heart, the ablation catheter 102 may be used to map and/or ablate myocardial tissue using the ring electrodes 118A, 118B, and 118C, the electrodes 122A, 122B, and 122C, and/or the tissue ablation electrode 120. In embodiments, the tissue ablation electrode 120 may be configured to apply ablation energy to myocardial tissue of the heart of a patient.

The catheter 102 may be steerable to facilitate navigating the vasculature of a patient or navigating other lumens. For example, the distal portion 116 of the catheter 102 may be configured to be deflected by manipulation of the actuator 112 to effect steering the catheter 102. In some instances, the distal portion 116 of the catheter 102 may be deflected to position the tissue ablation electrode 120 and/or the electrodes 122A, 122B, and 122C adjacent target tissue or to position the distal portion 116 of the catheter 102 for any other purpose. Additionally, or alternatively, the distal portion 116 of the catheter 102 may have a pre-formed shape adapted to facilitate positioning the tissue ablation electrode 120 and/or the electrodes 122A, 122B, and 122C adjacent a target tissue. For example, the preformed shape of the distal portion 116 of the catheter 102 may include a radial shape (e.g., a generally circular shape or a generally semi-circular shape) and/or may be oriented in a plane transverse to a general longitudinal direction of the catheter 102.

In various embodiments, the electrodes 122A, 122B, and 122C are circumferentially distributed about the tissue ablation electrode 120 and electrically isolated therefrom. The electrodes 122A, 122B, and 122C can be configured to operate in unipolar or bipolar sensing modes. In some embodiments, the plurality of electrodes 122A, 122B, and 122C may define and/or at least partially form one or more bipolar electrode pairs. For one or more bipolar electrode pairs, the signal generator 108 may drive one or more currents between one or more of the bipolar electrode pairs to facilitate determining a local impedance. Additionally, or alternatively, one or more bipolar electrode pairs may be configured to measure electrical signals corresponding to a local impedance and/or a sensed electrical activity (e.g., an electrogram (EGM) reading) of the myocardial tissue proximate thereto. Additionally, or alternatively, the catheter system 100 may include one or more sensors (e.g., force sensors, temperature sensors, optical sensors, ultrasound sensors, and/or other physiological sensors) (not shown) to facilitate measuring and/or processing electrical signals including, for example, a local impedance and/or sensed electrical activity of the myocardial tissue proximate thereto. In embodiments, the measured signals from the electrodes 122A, 122B, and 122C can be provided to the mapping processor 106 for processing as described herein. In embodiments, an EGM reading or signal from a bipolar electrode pair may at least partially form the basis of a contact assessment, ablation area assessment (e.g., tissue viability assessment), and/or an ablation progress assessment (e.g., a lesion formation/maturation analysis), as discussed below.

Various embodiments may include, instead of, or in addition to, an ablation catheter 102, a mapping catheter (not shown) that includes mapping electrodes such as, for example, the electrodes 122A, 122B, and 122C, but does not necessarily include a tissue ablation electrode 120. In embodiments, for example, a mapping catheter may be utilized for mapping while performing an ablation with a separate ablation catheter (e.g., the ablation catheter 102), or independently of performing tissue ablation. In other embodiments, more than one mapping catheter may be used to enhance the mapping data. Additionally or alternatively to the circumferentially spaced electrodes 122A, 122B, and 122C, the catheter 102 may include one or more forward facing electrodes (not shown). The forward facing electrodes may be generally centrally located within the tissue ablation electrode 120 and/or at an end of a tip of the catheter 102.

The tissue ablation electrode 120 may be any length and may have any number of the electrodes 122A, 122B, and 122C positioned therein and spaced circumferentially and/or longitudinally about the tissue ablation electrode 120. In some instances, the tissue ablation electrode 120 may have a length of between one (1) mm and twenty (20) mm, three (3) mm and seventeen (17) mm, or six (6) mm and fourteen (14) mm. In one illustrative example, the tissue ablation electrode 120 may have an axial length of about eight (8) mm.

In some cases, the plurality of electrodes 122A, 122B, and 122C may be spaced at any interval about the circumference of the tissue ablation electrode 120. In one example, the tissue ablation electrode 120 may include at least three electrodes 122A, 122B, and 122C equally or otherwise spaced about the circumference of the tissue ablation electrode 118 and at the same or different longitudinal positions along the longitudinal axis of the tissue ablation electrode 120. In some illustrative instances, the tissue ablation electrode 120 may have an exterior wall that at least partially defines an open interior region (not shown). The exterior wall may include one or more openings for accommodating one or more electrodes 122A, 122B, and 122C. Additionally, or alternatively, the tissue ablation electrode 120 may include one or more irrigation ports (not shown). Illustratively, the irrigation ports, when present, may be in fluid communication with an external irrigation fluid reservoir and pump (not shown) which may be used to supply fluid (e.g., irrigation fluid) to myocardial tissue to be or being mapped and/or ablated.

The RF generator 104 may be configured to deliver ablation energy to the ablation catheter 102 in a controlled manner in order to ablate the target tissue sites identified by the mapping processor 106. Ablation of tissue within the heart is well known in the art, and thus for purposes of brevity, the RF generator 104 will not be described in further detail. Further details regarding RF generators are provided in U.S. Patent 5,383,874. Although the mapping processor 106 and RF generator 104 are shown as discrete components, they can alternatively be incorporated into a single integrated device.

The RF ablation catheter 102 as described may be used to perform various diagnostic functions to assist the physician in an ablation treatment. For example, in some embodiments, the catheter 102 may be used to ablate cardiac arrhythmias, and at the same time provide real-time assessment of a lesion formed during RF ablation. Real-time assessment of the lesion may involve any of monitoring surface and/or tissue temperature at or around the lesion, reduction in the electrocardiogram signal, a drop in impedance, direct and/or surface visualization of the lesion site, and imaging of the tissue site (e.g., using computed tomography, magnetic resonance imaging, ultrasound, etc.). In addition, the presence of the electrodes within the RF tip electrode can operate to assist the physician in locating and positioning the tip electrode at the desired treatment site, and to determine the position and orientation of the tip electrode relative to the tissue to be ablated. As described herein, for example, embodiments include determining local impedance based on an analysis of a number of parameters (e.g., physiological parameters, device parameters, ablation parameters, and/or the like).

In operation and when the catheter 102 is within a patient and/or adjacent a target area, the catheter 102 may sense electrical signals (e.g., EGM signals) from the patient or target area and relay those electrical signals to a clinician (e.g., through the display of the RF ablation system 100). Electrophysiologists and/or others may utilize an EGM amplitude and/or EGM morphology to verify a location of the ablation catheter in a patient's anatomy, to verify viability of tissue adjacent the ablation catheter, to verify lesion formation in tissue adjacent the ablation catheter, and/or to verify or identify other characteristics related to the catheter 102 and/or adjacent target tissue or areas.

Based, at least in part, on its sensing capabilities, the catheter 102 may be utilized to perform various diagnostic functions to assist the physician in ablation and/or mapping procedures, as referred to above and discussed further below. In one example, the catheter 102 may be used to ablate cardiac arrhythmias, and at the same time provide real-time positioning information, real-time tissue viability information, and real-time assessment of a lesion formed during ablation (e.g., during RF ablation). Real-time assessment of the lesion may involve determining one or more local impedance metrics associated with the ablation site, such as, for example, an initial local impedance, a change in local impedance (e.g., an increase or decrease), an integral of an impedance signal over time, a derivative of an impedance signal over time, and/or the like.

"Real-time", as used herein and understood in the art, means during an action or process. For example, where one is monitoring local impedance metrics in real time during an ablation at a target area, the local impedance metrics are being monitored during the process of ablating at a target area (e.g., during or between applications of ablation energy). Additionally, or alternatively, the presence of electrodes 120A, 120B, and 120C at or about the tissue ablation electrode 120 and/or within the tip (e.g., at the distal tip) of the catheter 102 may facilitate allowing a clinician to locate and/or position the tissue ablation electrode 120 at a desired treatment site, to determine the position and/or orientation of the tissue ablation electrode relative to the tissue that is to be ablated or relative to any other feature.

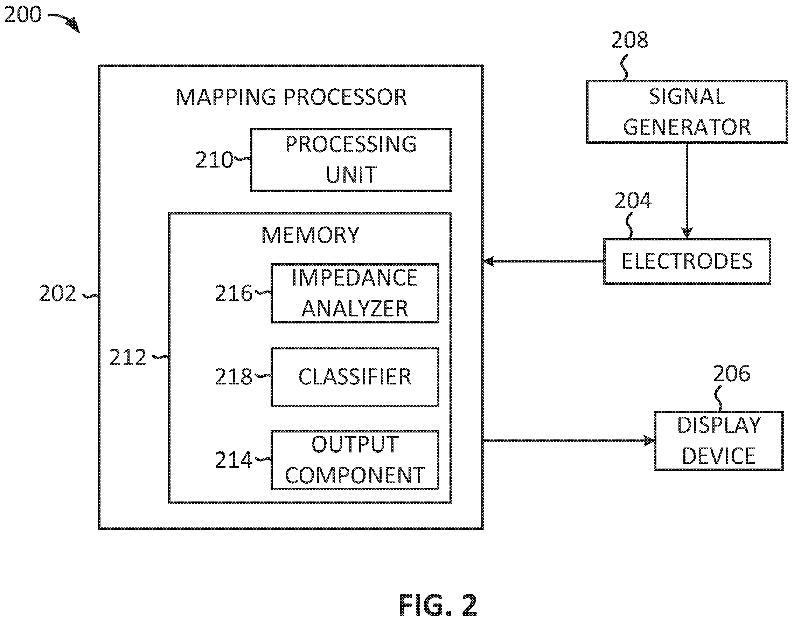

FIG. 2 depicts an illustrative mapping operating environment 200 in accordance with embodiments of the present invention. In various embodiments, a mapping processor 202 (which may be, or be similar to, mapping processor 106 depicted in FIG. 1) may be configured to detect, process, and record electrical signals associated with myocardial tissue via a catheter such as the ablation catheter 102 depicted in FIG. 1, a mapping catheter, and/or the like. In embodiments, based on these electrical signals, a clinician can identify the specific target tissue sites within the heart, and ensure that the arrhythmia causing substrates have been electrically isolated by the ablative treatment. The mapping processor 202 is configured to process signals from electrodes 204 (which may include, e.g., electrodes 122A, 122B, and 122C and/or ring electrodes 118A, 118B, and 118C depicted in FIG. 1), and to generate an output to a display device 206. A signal generator 208 may be configured to drive one or more currents to one or more of the electrodes 204 to facilitate determining a local impedance.

The display device 206 may be configured to present an indication of a tissue condition, effectiveness of an ablation procedure, and/or the like (e.g., for use by a physician). In some embodiments, the display device 206 may include electrocardiogram (ECG) information, which may be analyzed by a user to determine the existence and/or location of arrhythmia substrates within the heart and/or determine the location of an ablation catheter within the heart. In various embodiments, the output from the mapping processor 202 can be used to provide, via the display device 206, an indication to the clinician about a characteristic of the ablation catheter and/or the myocardial tissue being mapped.

In instances where an output is generated to a display device 206 and/or other instances, the mapping processor 202 may be operatively coupled to or otherwise in communication with the display device 206. In embodiments, the display device 206 may include various static and/or dynamic information related to the use of an RF ablation system (e.g., the RF ablation system 100 depicted in FIG. 1). For example, the display device 206 may present an image of the target area, an image of the catheter, and/or information related to EGMs, which may be analyzed by the user and/or by a processor of the RF ablation system to determine the existence and/or location of arrhythmia substrates within the heart, to determine the location of the catheter within the heart, and/or to make other determinations relating to use of the catheter and/or other catheters.

In embodiments, the display device 206 may be an indicator. The indicator may be capable of providing an indication related to a feature of the output signals received from one or more of the electrodes 204. For example, an indication to the clinician about a characteristic of the catheter and/or the myocardial tissue interacted with and/or being mapped may be provided on the display device 206. In some cases, the indicator may provide a visual and/or audible indication to provide information concerning the characteristic of the catheter and/or the myocardial tissue interacted with and/or being mapped. In embodiments, the visual indication may take one or more forms. In some instances, a visual color or light indication on a display 206 may be separate from or included on an imaged catheter on the display 206 if there is an imaged catheter. Such a color or light indicator may include a progression of lights or colors that may be associated with various levels of a characteristic proportional to the lesion size and/or another lesion characteristic. Alternatively, or in addition, an indicator indicating a feature of a characteristic may be provided in any other manner on a display and/or with any audible or other sensory indication, as desired. In embodiments, for example, a tactile indication may be provided such as, for example, by causing a handle of the catheter or other device to vibrate.

In some cases, a visual indication may be an indication on a display device 206 (e.g., a computer monitor, touchscreen device, and/or the like) with one or more lights or other visual indicators. In one example of an indicator, a color of at least a portion of an electrode of a catheter imaged on a screen of the display 206 may change from a first color (e.g., red or any other color) when there is poor contact between the catheter and tissue to a second color (e.g., green or any other color different than the first color) when there is good contact between the catheter and the tissue and/or when ablation may be initiated after establishing good contact. Additionally, or alternatively, in embodiments of an indicator, when a local impedance metric reaches or exceeds a threshold, a depicted color of an electrode on the imaged catheter may change colors to indicate a level of lesion maturation. In a similar manner, an indicator may be utilized to indicate a viability of tissue to be ablated. In the examples above, the changing color/light or changing other indicator (e.g., a number, an image, a design, etc.) may be located at a position on the display other than on the imaged catheter, as desired. According to embodiments, indicators may provide any type of information to a user. For example, the indicators discussed herein may be pass or fail type indicators showing when a condition is present or is not present and/or may be progressive indicators showing the progression from a first level to a next level of a characteristic.

According to embodiments, various components (e.g., the mapping processor 202 and/or the signal generator 208) of the operating environment 200, illustrated in FIG. 2, may be implemented on one or more computing devices. A computing device may include any type of computing device suitable for implementing embodiments of the disclosure. Although the components of a computing device are illustrated in FIG. 2 in connection with the mapping processor 202, it should be understood that the discussion herein regarding computing devices and components thereof applies generally to any number of different aspects of embodiments of the systems described herein that may be implemented using one or more computing devices. Examples of computing devices include specialized computing devices or general-purpose computing devices such "workstations," "servers," "laptops," "desktops," "tablet computers," "hand-held devices," and the like, all of which are contemplated within the scope of FIG. 2 with reference to various components of the operating environment 200.

In embodiments, a computing device includes a bus that, directly and/or indirectly, couples the following devices: a processing unit (e.g., the processing unit 210 depicted in FIG. 2), a memory (e.g., the memory 212 depicted in FIG. 2), an input/output (I/O) port, an I/O component (e.g., the output component 214 depicted in FIG. 2), and a power supply. Any number of additional components, different components, and/or combinations of components may also be included in the computing device. The bus represents what may be one or more busses (such as, for example, an address bus, data bus, or combination thereof). Similarly, in embodiments, the computing device may include a number of processing units (which may include, for example, hardware, firmware, and/or software computer processors), a number of memory components, a number of I/O ports, a number of I/O components, and/or a number of power supplies. Additionally any number of these components, or combinations thereof, may be distributed and/or duplicated across a number of computing devices.

In embodiments, the memory 210 includes computer-readable media in the form of volatile and/or nonvolatile memory and may be removable, nonremovable, or a combination thereof. Media examples include Random Access Memory (RAM); Read Only Memory (ROM); Electronically Erasable Programmable Read Only Memory (EEPROM); flash memory; optical or holographic media; magnetic cassettes, magnetic tape, magnetic disk storage or other magnetic storage devices; data transmissions; or any other medium that can be used to store information and can be accessed by a computing device such as, for example, quantum state memory, and the like.

In embodiments, the memory 210 stores computer-executable instructions for causing the processing unit 208 to implement aspects of embodiments of system components and/or to perform aspects of embodiments of methods and procedures discussed herein. Computer-executable instructions may include, for example, computer code, machine-useable instructions, and the like such as, for example, program components capable of being executed by one or more processors associated with a computing device. Examples of such program components include an impedance analyzer 216 and a classifier 218. Program components may be programmed using any number of different programming environments, including various languages, development kits, frameworks, and/or the like. Some or all of the functionality contemplated herein may also be implemented in hardware and/or firmware.

According to embodiments, the impedance analyzer 216 and classifier 218 may be used for determining a lesion characteristic. For example, the impedance analyzer 216 and/or classifier 218 may be configured to determine local impedance characteristics based on electrical signals received from one or more electrodes 204. In embodiments, the signal generator 208 may be configured to drive one or more currents through a first set of electrodes 204 and the mapping processor 202 may be configured to receive electrical signals measured by a second set of electrodes 204, analyze the electrical signals received to determine one or more local impedance metrics, and determine one or more lesion characteristics based on the one or more local impedance metrics. In embodiments, the second set of electrodes may include at least one different electrode than the first set of electrodes.

In embodiments, local impedance refers to an impedance measured between two or more electrodes disposed adjacent a target location. For example, in embodiments, a local impedance may be measured between two electrodes that are disposed on a distal end of an ablation catheter, based on a signal that is generated using two or more other electrodes disposed on the catheter. In embodiments, multiple measurements of local impedance may be taken using multiple combinations of electrodes. For example, in embodiments, a first signal may be generated using a first pair (e.g., where the first set of electrodes includes two electrodes) of electrodes and the impedance between the two electrodes of the first pair may be measured using a second pair of electrodes. In embodiments, a second signal may be generated using a third pair of electrodes and the impedance measured by using a fourth set of electrodes, and so on. When multiple impedance measurements are collected, embodiments include selecting one or more of the multiple measurements to analyze for determining one or more lesion characteristics. If one impedance measurement is selected for analysis, embodiments include using one or more additional local impedance measurements as a check against the first measurement, as training data to train the classifier 218, and/or the like.

In some instances, lesion characteristics may be further analyzed in a meaningful manner in real-time (e.g., during a typical electrophysiology procedure) by determining a local impedance associated with the catheter and determining, based on the local impedance, a characteristic of lesion maturation. In some embodiments, one or more sensors (e.g., a force sensor, temperature sensor, ultrasound sensor and/or other physiological sensor) may be used to facilitate determining a local impedance associated with the catheter and/or a characteristics of lesion maturation. In embodiments, determining the local impedance may include performing a machine-learning algorithm and/or other modeling algorithm with the mapping processor 202 and/or other processor. According to embodiments, real-time monitoring may be facilitated using multiplexing techniques, phase differentiation techniques, frequency filtering techniques, and/or the like. In embodiments, the signal generator may be configured to generate an electrical signal that is distinguishable from RF energy being delivered for ablation (and, e.g., that doesn't interfere with the RF energy, and/or vice-versa) such as, for example, by generating an electrical signal that has a different frequency than the RF energy signal.

The output component 214 may be configured to provide an output to the display device 206, where the output includes the determined feature (e.g., an indication of a lesion characteristic). For example, the display device 206 may be configured to indicate a relative change in the local impedance during a period of time, an estimated lesion size and/or depth, likelihood of occurrence of a steam pop, and/or the like.

The illustrative operating environment 200 shown in FIG. 2 is not intended to suggest any limitation as to the scope of use or functionality of embodiments of the present disclosure. Neither should it be interpreted as having any dependency or requirement related to any single component or combination of components illustrated therein. Additionally, any one or more of the components depicted in FIG. 2 may be, in embodiments, integrated with various ones of the other components depicted therein (and/or components not illustrated), all of which are considered to be within the ambit of the present disclosure. For example, the impedance analyzer 216 may be integrated with the classifier 218, the classifier 218 may be included in the impedance analyzer 216, and/or the like. In embodiments, any number of components such as those depicted in FIG. 2 may be utilized to estimate lesion maturation, as described herein.

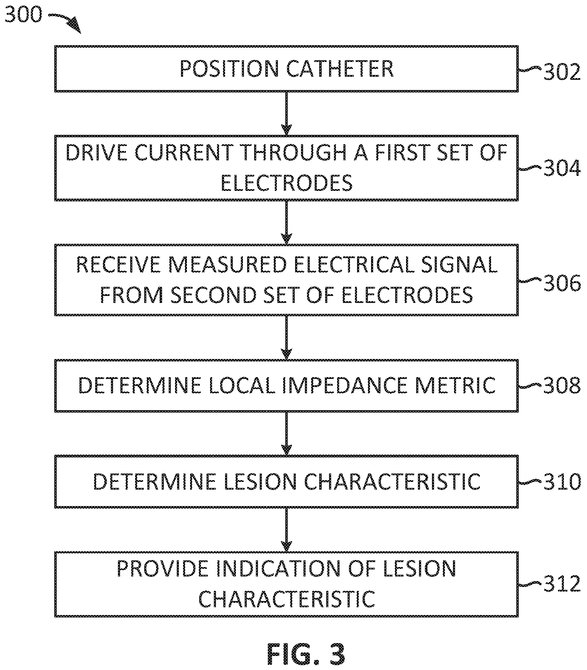

As described above, in embodiments, a mapping processor (e.g., the mapping processor 106 depicted in FIG. 1 and/or the mapping processor 202 depicted in FIG. 2) may utilize local impedance measurements, and, in embodiments, any number of other parameters, to analyze lesion maturation. FIG. 3 depicts an illustrative method 300 of analyzing lesion maturation of an ablation lesion, in accordance with embodiments of the subject matter described herein. In the illustrative method 300, a distal portion of a catheter (e.g., the catheter 102 depicted in FIG. 1, a mapping catheter, and/or the like) may be positioned at a location proximate a target area or target tissue (block 302). A signal generator (e.g., the signal generator 108 depicted in FIG. 1 and/or the signal generator 208 depicted in FIG. 2) may be configured to drive one or more currents through a first set of electrodes (block 304). A mapping processor (e.g., the mapping processor 106 depicted in FIG. 1 and/or the mapping processor 202 depicted in FIG. 2) may receive electrical signal measurements from a second set of electrodes adjacent a target area or tissue (block 306). Illustratively, the signals measured by the electrodes of the catheter may be used to determine a local impedance metric (block 308).

A set of electrodes may include one or more electrodes. In embodiments, any number of different combination of electrodes (e.g., ring electrodes, pin electrodes, ablation electrodes, etc.) may be used to generate a signal and/or measure impedance based on the generated signal. In embodiments, the first set of electrodes may be the same as the second set of electrodes or the first and second sets may differ by at least one electrode. According to embodiments, all different combinations of pairs of electrodes may be used to respectively generate signals and obtain impedance measurements based on those signals. According to embodiments, multiple impedance measurements may be used to compare with other impedance measurements. In embodiments, multiple impedance measurements may be aggregated (e.g., using statistical or other mathematical methods) to determine an impedance metric (e.g., an average impedance, etc.). For example, impedance measurements from various electrode sets may be assigned corresponding weights (e.g., based on electrode location, signal quality, etc.), and a weighted average, or other linear or nonlinear combination of weighted impedance measurements may be determined and used to determine lesion characteristics. Multiple impedance measurements and/or generated signals may be multiplexed (e.g., time-based, code-based, frequency-based, etc.) to facilitate multiple impedance measurements within a relatively small window (that is, for example, multiple impedance measurements may be obtained at approximately the same point in time).

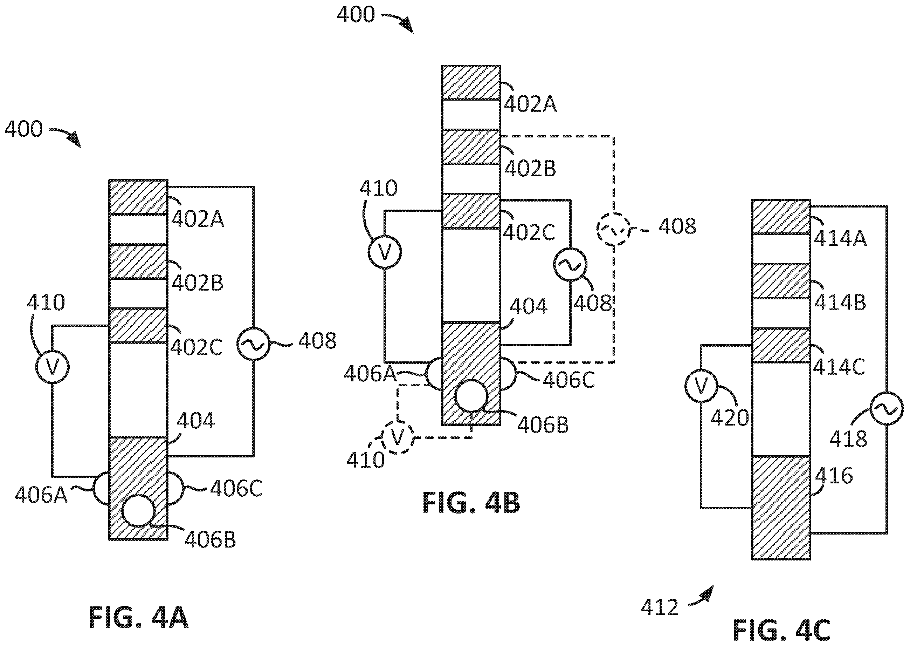

FIGS. 4A-4C are schematic diagrams depicting illustrative electrode arrangements for determining local impedance, in accordance with embodiments of the subject matter disclosed herein. As shown in FIGS. 4A and 4B, an illustrative ablation catheter 400 includes three ring electrodes 402A, 402B, and 402C, an RF ablation electrode 404, and three mapping electrodes 406A, 406B, and 406C. As shown in FIG. 4A, a first electrode arrangement includes a first set of electrodes 402A and 404 between which a current 408 is driven and a second set of electrodes 402C and 406A used to obtain a measurement 410 of the electrical signal generated by the first set of electrodes. In FIG. 4B, a second electrode arrangement is depicted in which the current 408 is driven between the ring electrode 402C and the ablation electrode 404, while a measurement 410 is obtained using the ring electrode 402B and the pin electrode 406A. An alternative arrangement is also shown in FIG. 4B, in which the current 408 is driven between the ring electrode 402B and the pin electrode 406C, and in which a corresponding impedance measurement 410 is obtained using the pin electrode 406A and the pin electrode 406B.

In FIG. 4C, an ablation catheter 412 includes a number of ring electrodes 414A, 414B, and 414C, and an ablation (e.g., RF) electrode 416, but no pin electrodes disposed on the tip. In embodiments, as shown, an electrode arrangement may include a first set of electrodes 414A and 416 that is used for generating a signal 418, and a second set of electrodes 414C and 416 that is used for obtaining a local impedance measurement 420 based on that signal 418. As with aspects of embodiments depicted in FIGS. 4A and 4B, any number of different combinations of the electrodes 414A, 414B, 414C, and 416 may be used to respectively generate signals and obtain local impedance measurements based on those signals.

The illustrative catheters 400 and 412, and electrode arrangements shown in FIGS. 4A-4C are not intended to suggest any limitation as to the scope of use or functionality of embodiments of the present disclosure. Neither should they be interpreted as having any dependency or requirement related to any single component or combination of components illustrated therein. Additionally, any one or more of the components and/or arrangements depicted in FIGS. 4A-4C may be, in embodiments, integrated with various ones of the other components and/or arrangements depicted therein (and/or components not illustrated), all of which are considered to be within the ambit of the present disclosure.

As is further shown in FIG. 3, embodiments of the illustrative method 300 further include determining a lesion characteristic (block 310) and providing an indication of the lesion characteristic (block 312). According to embodiments, the mapping processor determines a lesion characteristic based on a local impedance metric (e.g., an initial local impedance, a change in local impedance (e.g., an increase or decrease), an integral of an impedance signal over time, a derivative of an impedance signal overtime, etc.). In embodiments, the mapping processor may also utilize any number of other parameters in determining the lesion characteristic. For example, in embodiments, the mapping processor may use a measure of catheter stability, a measure of RF generator impedance, a measure of EGM attenuation, a measure of RF dose (e.g., power and duration), a measure of contact force, a measure of temperature, a measure of an optical property, an ultrasound measure, and/or the like. As indicated herein, a lesion characteristic may include, for example, an existence of a lesion, a size of a lesion (e.g., an amount of tissue surface area occupied by the lesion), a depth of a lesion, a likelihood of an occurrence of a steam pop (e.g., a calculated probability that, given a particular set of circumstances, a steam pop will occur within a specified time window), and/or the like.

To determine the lesion characteristic, the mapping processor may be configured to utilize a classifier and/or other machine-learning algorithm. In embodiments, multiple classifiers may be used in parallel, in series, and/or in any number of other integrated manners. According to embodiments, the classifier may include a decision-tree algorithm, a support vector machine (SVM), and, in embodiments, any number of other machine-learning techniques. According to embodiments, supervised and/or unsupervised learning may be employed to increase the accuracy and efficiency of the algorithms used for determining lesion characteristics over time. Neural networks, deep learning, and/or other multi-variate classification techniques may be utilized. In embodiments, for example, using decision trees may facilitate more efficient computation, as decision trees can be structured to group relevant metrics and parameters, while excluding others. In some embodiments, systems and/or methods described herein may be configured to determine a likelihood of an occurrence of a steam pop (e.g., in addition to, or in lieu of, other lesion characteristics). In embodiments of such cases, computational burdens may be reduced from those of conventional systems by using a binary classifier (e.g., a decision tree configured to facilitate a binary classification, etc.).

According to embodiments, a level of one or more lesion characteristics may be represented and/or monitored via the determined local impedance. The one or more lesion characteristics may include, for example, contact force between the catheter and a target area (e.g., a target tissue or other target area), viability of a target area, ablation progress (e.g., lesion maturation or other metric of ablation progress), conduction characteristics of a target area, a likelihood of an occurrence of a steam pop (e.g., in view of a particular set of circumstances that may, for example, represent the particular state of the ablation procedure as determined at the time of measurement), and/or the like. In embodiments, one or more of these or other characteristics can be represented and/or monitored, as described herein, in real time, for example, while positioning the distal portion of the catheter proximate the target area, while mapping a target area or other object, while applying ablation energy to a target area, and/or while performing any other action with the catheter. The level of the one or more of the characteristics may be displayed visually on a display, may be indicated by an audible indicator, or may be indicated in any other manner. In embodiments, the indication may include a map (e.g., a voltage map, a frequency spectral map, etc.), a light indicator, a waveform, and/or the like.

Embodiments of the systems and methods described herein include closed-loop systems in which determined lesion characteristics may trigger an action taken by the system. For example, in embodiments, a mapping processor and/or RF generator may be configured to discontinue an RF ablation procedure (e.g., by discontinuing delivery of RF energy via the ablation electrode) in response to determining that a lesion size, depth, or other characteristic has reached or exceeded a specified threshold. Additionally, or alternatively, a mapping processor and/or RF generator may be configured to discontinue an RF ablation procedure in response to determining that a likelihood of occurrence of a steam pop reaches or exceeds a specified threshold. In embodiments, a mapping processor and/or RF generator may be configured to adjust an ablation parameter (e.g., frequency, amplitude, etc.) in response to determination of a lesion characteristic (e.g., a lesion characteristic that indicates that the target tissue is diseased). In embodiments, for example, in response to determining that a likelihood of an occurrence of a steam pop reaches or exceeds a threshold, the mapping processor and/or RF generator may be configured to decrease the RF power being delivered and, in embodiments, may accompany this decrease with a notification to the clinician that a steam pop may occur and, for example, that the clinician should discontinue (at least temporarily or in a specific location) the ablation procedure.

Various modifications and additions can be made to the exemplary embodiments discussed without departing from the scope of the present disclosure. For example, while the embodiments described above refer to particular features, the scope of this disclosure also includes embodiments having different combinations of features and embodiments that do not include all of the described features. For example, embodiments may include combining assessment of local impedance with other techniques (e.g., contact force techniques, etc.), temperature measurements, ultrasound techniques, and/or the like, to enhance determinations of lesion characteristics. Additionally, or alternatively, embodiments may include injecting current and creating a field from an ablation catheter electrode to a separate intracardiac catheter, such as a catheter positioned in the coronary sinus, or pulmonary vein, or other region in the heart. Embodiments may include injecting current between electrodes on an ablation catheter and sensing the corresponding voltage from an electrode on the ablation catheter to an electrode on a separate intracardiac catheter.

## Claims

1. An electrophysiology system, comprising:
a catheter (102) including:
a flexible catheter body (114) having a distal portion (116); and
a plurality of electrodes disposed on the distal portion (116), the plurality of electrodes including:
a radiofrequency (RF) ablation electrode (404);
first, second and third mapping electrodes (406A, 406B, 406C) positioned within the RF ablation electrode; and
a plurality of ring electrodes (402A, 402B, 402C) located proximally of the RF ablation electrode (404) and including a first ring electrode (402A) located proximally to a distal end of the catheter (102) , an intermediate second ring electrode (402B) positioned distally of the first ring electrode (402A), and a distal ring electrode (402C) located distally of the second ring electrode (402B);
a signal generator (108) configured to generate an electrical signal by driving one or more currents (408) between a first set of the plurality of electrodes defined by the RF ablation electrode (404) and the first ring electrode (402A), wherein a second set of the plurality of electrodes defined by one of the mapping electrodes (406A, 406B, 406C) and the distal ring electrode (402C) is configured to obtain an impedance measurement based on the electrical signal; and
a mapping processor (106) configured to:
receive the impedance measurement from the second set of electrodes;
determine at least one impedance metric; and
determine at least one lesion characteristic based on the at least one impedance metric.

2. The system of claim 1, wherein the at least one lesion characteristic comprises at least one of an existence of the lesion, a depth of the lesion, and/or a size of the lesion.

3. The system of any of claims 1-2, wherein the at least one impedance metric comprises at least one of an initial impedance, an impedance drop, a derivative of an impedance signal over a period of time, and an integral of an impedance signal over time.

## Patentansprüche

1. Elektrophysiologiesystem, umfassend:
einen Katheter (102), der einschließt:
einen flexiblen Katheterkörper (114) mit einem distalen Abschnitt (116); und
eine Mehrzahl von Elektroden, die an dem distalen Abschnitt (116) angeordnet sind, wobei die Mehrzahl von Elektroden einschließt:
eine Funkfrequenz(FF)-Ablationselektrode (404);
eine erste, eine zweite und eine dritte Mapping-Elektrode (406A, 406B, 406C), die innerhalb der FF-Ablationselektrode positioniert sind; und
eine Mehrzahl von Ringelektroden (402A, 402B, 402C), die proximal von der FF-Ablationselektrode (404) angeordnet sind und eine proximal zu einem distalen Ende des Katheters (102) angeordnete erste Ringelektrode (402A), eine distal zu der ersten Ringelektrode (402A) angeordnete mittlere, zweite Ringelektrode (402B) und eine distal zu der zweiten Ringelektrode (402B) angeordnete distale Ringelektrode (402C) umfassen;
einen Signalgenerator (108), der ausgebildet ist, durch Fließen lassen eines oder mehrerer Ströme (408) zwischen einem ersten Satz von der Mehrzahl von Elektroden, der von der FF-Ablationselektrode (404) und der ersten Ringelektrode (402A) definiert wird, ein elektrisches Signal zu erzeugen, wobei ein zweiter Satz von der Mehrzahl von Elektroden, der von einer der Mapping-Elektroden (406A, 406B, 406C) und der distalen Ringelektrode (402C) definiert wird, ausgebildet ist, um eine Impedanzmessung auf Basis des elektrischen Signals zu erhalten; und
einen Mapping-Prozessor (106), der ausgebildet ist zum:
Empfangen der Impedanzmessung von dem zweiten Satz von Elektroden;
Bestimmen mindestens einer Impedanzmetrik; und
Bestimmen mindestens einer Läsionseigenschaft auf Basis der mindestens einen Impedanzmetrik.

2. System nach Anspruch 1, wobei die mindestens eine Läsionseigenschaft mindestens eines von einem Vorhandensein der Läsion, einer Tiefe der Läsion und/oder einer Größe der Läsion umfasst.

3. System nach einem der Ansprüche 1-2, wobei die mindestens eine Impedanzmetrik mindestens eines von einer Anfangsimpedanz, einem Impedanzabfall, einer Ableitung eines Impedanzsignals im Zeitverlauf und einem Integral eines Impedanzsignals im Zeitverlauf einschließt.

## Revendications

1. Système d'électrophysiologie, comprenant :
un cathéter (102) incluant :
un corps de cathéter souple (114) comportant une section distale (116) ; et
une pluralité d'électrodes disposées sur la section distale (116), la pluralité d'électrodes incluant :
une électrode d'ablation radiofréquence (RF) (404) ;
des première, deuxième et troisième électrodes de cartographie (406A, 406B, 406C) positionnées à l'intérieur de l'électrode d'ablation RF ; et
une pluralité d'électrodes en anneau (402A, 402B, 402C) localisées de façon proximale par rapport à l'électrode d'ablation RF (404) et incluant une première électrode en anneau (402A) localisée de façon proximale par rapport à une extrémité distale du cathéter (102), une seconde électrode en anneau (402B) intermédiaire positionnée de façon distale par rapport à la première électrode en anneau (402A) et une électrode en anneau distale (402C) localisée de façon distale par rapport à la seconde électrode en anneau (402B) ;
un générateur de signal (108) configuré pour générer un signal électrique en pilotant un ou plusieurs courants (408) entre un premier ensemble de la pluralité d'électrodes qui est défini par l'électrode d'ablation RF (404) et par la première électrode en anneau (402A), dans lequel un second ensemble de la pluralité d'électrodes qui est défini par l'une des électrodes de cartographie (406A, 406B, 406C) et par l'électrode en anneau distale (402C) est configuré pour obtenir une mesure d'impédance sur la base du signal électrique ; et
un processeur de cartographie (106) configuré pour :
recevoir la mesure d'impédance en provenance du second ensemble d'électrodes ;
déterminer au moins une métrique d'impédance ; et
déterminer au moins une caractéristique de lésion sur la base de l'au moins une métrique d'impédance.

2. Système selon la revendication 1, dans lequel l'au moins une caractéristique de lésion comprend au moins une caractéristique parmi une existence de la lésion, une profondeur de la lésion et/ou une dimension de la lésion.

3. Système selon l'une quelconque des revendications 1 et 2, dans lequel l'au moins une métrique d'impédance comprend au moins une valeur parmi une impédance initiale, une chute d'impédance, une dérivée d'un signal d'impédance sur une période de temps et une intégrale d'un signal d'impédance sur le temps.
